# EUROPEAN PATENT APPLICATION

(11) **EP 4 592 404 A1**
(43) Date of publication of application: **30.07.2025**
(21) Application number: 24154360.2
(22) Date of filing: 29.01.2024
(51) Int. Cl.: C12Q 1/6886, G01N 33/574

(54) **ASAH1 AS A BIOMARKER FOR DETERMINING THE RISK FOR RECURRENCE OF GLIOBLASTOMA MULTIFORME IN A SUBJECT**

(71) Applicant: Albert-Ludwigs-Universität Freiburg, 79098 Freiburg (DE); Philipps-Universität Marburg, 35037 Marburg (DE)
(72) Inventor: Schelling, Oliver, 4052 Basel (CH); Cosenza-Contreras, Miguel de Jesus, 79115 Freiburg (DE); Werner, Tilman, 79106 Freiburg (DE); Schäfer, Agnes, 57074 Siegen (DE); Bartsch, Jörg Walter, 33617 Bielefeld (DE)
(74) Representative: Krauss, Jan

(57) **Abstract**

The present invention relates to a method for determining the risk for recurrence of glioblastoma multiforme (GBM) in a subject, comprising determining the expression level of acid ceramidase (*ASAH1*) in a biological sample obtained from the subject, wherein an increase of *ASAH1* expression level in the biological sample as determined when compared to a control sample indicates a lower risk for recurrence of GBM in the subject. Based on the risk for recurrence as determined, the invention allows for providing a prognosis for recurrence of glioblastoma multiforme, monitoring the status of GBM in a subject, identifying a subject having GBM in need of adjuvant therapy and/or identifying a candidate compound for modulating the time for recurrence of GBM. The present invention also provides an array and a kit for the above-mentioned methods.

## Description

### Field of the invention

The present invention relates to a method for determining the risk for recurrence of glioblastoma multiforme (GBM) in a subject, comprising determining the expression level of acid ceramidase (*ASAH1*) in a biological sample obtained from the subject, wherein an increase of *ASAH1* expression level in the biological sample as determined when compared to a control sample indicates a lower risk for recurrence of GBM in the subject. Based on the risk for recurrence as determined, the invention allows for providing a prognosis for recurrence of glioblastoma multiforme, monitoring the status of GBM in a subject, identifying a subject having GBM in need of adjuvant therapy and/or identifying a candidate compound for modulating the time for recurrence of GBM. The present invention also provides an array and a kit for the above-mentioned methods.

### Background of the invention

Each year, about 5-6 cases out of 100,000 people are diagnosed with primary malignant brain tumors, of which about 80% are malignant gliomas. Gliomas is the most common group of primary brain tumors. According to World Health Organization (WHO) malignant gliomas are subcategorized into grade III/IV tumors such as anaplastic astrocytoma, anaplastic oligodendroglioma, anaplastic oligoastrocytoma and anaplastic ependymomas, as well as grade IV/IV tumors, as glioblastoma multiforme (GBM). Glioblastoma multiforme WHO IV is the most prevalent, malignant, and aggressive type of brain tumor and accounts for more than half of the malignant gliomas. The WHO categorization is based on certain pathological features, such as nuclear atypia, mitotic activity, vascular proliferation, necrosis, proliferative potential and features clinical course and treatment outcome.

The current treatment involves maximal surgical resection additional to radiotherapy plus concomitant and adjuvant temozolomide or carmustin wafers as standard care in patients younger than 70 years old with newly diagnosed GBM. Despite this treatment, the median survival rate of 15 months remains discouragingly low, with a mean survival rate of only 3.3% at 2 years and 1.2% at 3 years. The recurrence of the GBM tumors seems to be the rule leading to an infiltration of functional areas of the brain diffusely. Therapeutically resistant glioblastoma or glioblastoma stem-like cells (GSCs) are hypothesized to cause the recurrence. Recurrent GBM lacks a standard treatment and only 20-30% of the recurrent cases are considered feasible for a second surgery due to limited accessibility. The identification of molecular signatures as diagnostic and/or prognostic markers allows a potential early detection of the onset of recurrence of GBM.

Acid ceramidase (ASAH1) is a lysosomal cysteine amidase that catalyzes the transformation of ceramide into sphingosine and free fatty acid. Subsequently, sphingosine kinase 1 (SPHK1) or 2 (SPHK2) phosphorylate sphingosine forms the tumor promoter, sphingosine-1-phosphate (S1P). ASAH1, initially discovered in rat brain homogenates and further characterized and purified from human urine in 1995, has a molecular weight of 53-55 kDa, depending on the extent of glycosylation. It is composed of α- and β-subunits, 13 kDa and 40 kDa in size respectively, that are linked by disulfide bonds. ASAH1 has been linked to multiple cancers, such as melanoma, acute myeloid leukemia (AML), and colon and prostate cancers.

Doan *et al.,* 2017b discloses an association of acid ceramidase (ASAH1) with glioblastoma. CD133+ glioblastoma cancer stem cells (GSCs) express significantly higher ASAH1 compared to CD133- GSCs and serum-cultured glioblastoma cell lines. ASAH1 inhibition increases cellular ceramide level and induces apoptosis. A serum-cultured glioblastoma cell line and three different patient-derived glioblastoma stem-like cancer cell lines were efficiently killed, through apoptosis, by three different ASAH1 inhibitors with IC50's ranging from 11-104 µM. Herein, ASAH1 is identified as a *de novo* glioblastoma drug target, and ASAH1 inhibitors are shown to be highly effective for glioblastoma treatment by targeting specifically glioblastoma cancer stem cells.

Doan *et al.,* 2017a additionally discloses that GBMs acquire resistance to radiation via upregulation of acid ceramidase (ASAH1) and sphingosine-1-phosphate (Sph-1P). Moreover, inhibition of ASAH1 and Sph-1P, either with humanized monoclonal antibodies, small molecule drugs (i.e. carmofur), or a combination of both, led to suppression of GBM cell growth. These results suggest that ASAH1 and Sph-1P may be excellent targets for the treatment of new GBMs and recurrent GBMs, especially since the latter overexpresses ASAH1.

For a better understanding of the correlation between the *ASAH1* expression and GBM cell growth, the *ASAH1* gene expression was evaluated in different available patient mRNA datasets. High ASAH1 mRNA correlated with a shorter median survival in all three databases for IDH1-wildtype GBM patients. Elevated ASAH1 mRNA was also significantly associated with worse survival for all glioma patients (GBM and low-grade gliomas).

Nguyen et al., 2018 discloses that the regulation of ASHA1 may have been altered in GBM, allowing ASAH1 to be secreted into the interstitial tissues, leading to a transfer of malignant potential from GBM ASAH1-secreting cells to nearby healthy cells. ASAH1 may decrease the survival of a patient from a glioblastoma tumor by enhancing the survival of CD133+ cells, therefore producing resistance to common anticancer therapies. The secretion of ASAH1 was also increased after radiation conferring radioresistance to GBM cells. A more water-soluble, potent derivative of carmofur may be used for inhibiting ASAH1 and as a new drug for regulation of the growth of GBM cells.

Hawkins et al, 2022 discloses the role of ASAH1 in GBM migration. The balance between ceramides and sphingosine-1-phosphate contributes to the ability of GBM cells to migrate or invade and an *ASAH1* expression also correlates with genes associated with migration and focal adhesion. An analysis of ASAH1 expression data suggests that ASAH1 regulates GBM cell migration through PI3K-AKT-mTOR signaling. The authors suggest that the inhibition of migration of cancer cells by blocking ASAH1 may be a new therapy for metastatic brain tumors.

KR20170121470A discloses the discovery of six mesenchymal tumor-associated macrophages (MES-TAM) signature genes, CFI, LAMB-1, DCBLD2, ADAMTS14, MMP9, and THY1, which are related to the actual induction of mesenchymal glioblastoma and can be used to predict the prognosis for glioblastoma recurrence. A diagnostic method is disclosed for diagnosing or predicting prognosis of glioblastoma comprising primers for measuring the gene expression from the above signature genes through qPCR. Additionally, a kit is disclosed which includes the primers for measuring the gene expression levels and contains essential elements for the performance of ELISA to detect the diagnostic markers which form an antigen-antibody complex.

CN108753984A discloses the risk of postoperative recurrence of malignant glioma, especially the recurrence of distant metastasis, by detecting the expression level of miRNA in cerebrospinal fluid exosomes. A biomarker combination is disclosed for predicting or diagnosing the postoperative recurrence of brain malignant glioma. The biomarker combination includes miR-10b, miR-485-5p, EGFRvII, and VEGF-A. Exosomes are isolated from an extraction of cerebrospinal fluid from patients with glioma and used as RNA template for the reverse transcription to cDNA. Specific primers are used for detecting the expression levels of the various biomarkers through real-time PCR. Also disclosed is a kit for the application of the biomarker combination.

WO2017/158358A1 relates to methods for selecting a treatment for a subject with cancer, predicting responsiveness of a subject with cancer to therapeutic agents including MAPK, EMT and SRC pathway inhibitors and determining clinical prognosis based on assessing the expression level of biomarkers. Three molecular subgroups of High grade serous ovarian cancer (HGSOC), an Angiogenesis subgroup (HGS 1), an Immune subgroup (HGS2) and an angioimmune subgroup (HGS3) were defined using gene expression data from 265 HGSOC samples obtained from treatment of subjects who were treated with carboplatin plus paclitaxel or carboplatin only Standard of Care (SoC) chemotherapy. It was demonstrated that the MAPK pathway is a mechanism of innate and acquired resistance to SoC cisplatin-based therapy in HGSOC and that the use of anti-angiogenic targeted agents work better in second line trials after primary cisplatin treatment. The cancer of the subject whose prognosis is determined may be a glioblastoma.

CN107782895A discloses a urine protein marker for brain glioma and application thereof in monitoring tumor treatment effects. The disclosure particularly relates to application of a glioma urea protein maker in monitoring treatment effects of a patient with brain glioma. The urine protein marker is selected from thrombospondin-4, collagen alpha-1(IV) chain, interleukin-1 receptor antagonist, ribonuclease Inhibitor, N-acetylmuramoyl-L-alanine amidases, fibronectin-1, polymeric immunoglobulin receptor, cathepsin D, cystatin-C, aminopeptidase N, dipeptidyl peptidase 1, beta-glucuronic acid, lysosome Pro-X carboxypeptidase, nectin-2, selenium-binding protein 1, programmed cell death protein 1 ligand 2, acid ceramidase, napsin-A, lysosome-associated membrane protein 1, glutamyl aminopeptidase, gamma-glutamyl hydrolase and the like. By using the urine protein marker, as an identifying reagent, it is possible to effectively determine postoperative treatment effects for a patient.

All prior art relating to an *ASAH1* expression in glioblastoma multiforme (GBM) patients discloses that an increase of *ASAH1* expression leads to a worse survival rate for all GBM patients. The known biomarkers for determining the risk of glioblastoma recurrence either do not relate to *ASAH1* expression in GBM patients or do not indicate that an increase of *ASAH1* expression leads to a lower risk for recurrence of GBM.

Despite all above progress, the prognosis for a longer survival rate of GBM in a patient remains poor. Therefore, there is still a need to provide novel diagnostic tools to assess tumor aggressiveness and predict each patient's therapeutic needs shortly and directly after surgery, respectively.

Consequently, the object of the present invention is to provide these diagnostic tools, and to develop improved respective anti-tumor therapies and strategies. Other objects and advantages of the present invention will become apparent to the person skilled in the art when studying the following more detailed description of the present invention, including the Figures and examples.

According to a first aspect of the present invention the above object is solved by providing a method for determining the risk for recurrence of glioblastoma multiforme (GBM) in a subject, comprising determining the expression level of acid ceramidase (*ASAH1*) in a biological sample obtained from the subject, wherein an increase of *ASAH1* expression level in the biological sample as determined when compared to a control sample indicates a lower risk for recurrence of GBM in the subject.

The inventors identified acid ceramidase (*ASAH1)* as a potential biomarker of early recurrence of GBM by analyzing the large-scale differential abundance of proteins in biological samples obtained from the subject at the time of the initial (iGBM) and recurrent glioblastoma multiforme (rGBM) tumor surgery. The tumor tissue was obtained from a group of 11 patients and stored fresh frozen for the analysis via liquid chromatography mass spectrometry (LC-MS/MS). The proteomics analysis yielded in the identification of 5,954 proteins including 3,228 proteins in every of the three biological replicates of the tumor samples.

The inventors also identified that an increase of *ASAH1* expression level in the biological samples indicates a lower risk for recurrence of GBM in the subject. This was a new finding for the *ASAH1* gene expression in glioblastoma multiforme. All the research data based on *ASAH1* gene expression in glioblastoma multiforme (GBM) patients discloses that an increase of *ASAH1* expression leads to a worse survival rate for all GBM patients. Therefore, the teaching of the prior art leads away from the present invention. Based on the prior art, a person skilled in the art would not be prompted to use an elevated *ASAH1* gene expression for a biomarker for a lower risk for recurrence of glioblastoma multiforme, consequently enabling a faster diagnosis for the recurrence of GBM and an adequate treatment to prevent the recurrence of GBM.

For the analysis of the proteomic data collected via LC-MS/MS, an additional sparse least square discriminant analysis (sPLS-DA) of the proteomics data was used to differentiate between initial GBM and recurrent GBM tumor samples. sPLS-DA indicated the quantitative differences between the two tumor stages. After further analysis, the inventors identified acid ceramidase (*ASAH1)* as a potential biomarker for the risk for recurrence of glioblastoma multiforme in a subject, preferably shortly after initial surgery of the glioblastoma tumor.

Preferred is the method for determining the risk for recurrence of glioblastoma multiforme (GBM) in a subject according to the present invention, wherein the biological sample is selected from the group consisting of blood, plasma, serum, urine, cerebral spinal fluid, synovial fluid, tumor tissue, and dissociated tumor cells. Particularly preferred is a biological sample selected from the group consisting of blood, plasma, and serum.

Further preferred is the method for determining the risk for recurrence of glioblastoma multiforme (GBM) in the subject according to the present invention, wherein the biological samples are obtained from the subject shortly after GBM tumor removal, preferably after the initial GBM tumor removal.

According to the present invention, any method is suitable to detect the expression level of acid ceramidase (*ASAH1*) known to the person skilled of art. Preferred is a method according to the present invention, wherein the detection of the expression level of acid ceramidase (*ASAH1*) in the biological sample comprises a method measuring the mRNA or protein level selected from at least one of ELISA, immunohistochemical (IHC) staining, proteolysis, bicinchoninic acid assay, qPCR and mass spectrometry, preferably comprising a label-free quantitation (LFQ).

In context of the present invention, the immunohistochemical staining included the following steps, 2 µm tissue sections were deparaffinized and subjected to heat-induced antigen retrieval. Tissue sections were then stained using the following steps: incubation in H₂O₂ for 5 minutes, with primary antibodies for 1 hour, with mouse/rabbit linker (15 minutes), with horseradish peroxidase and secondary antibody for 20 minutes and final incubation with 3, 3'-diaminobenzidine for 10 minutes. Sections were then counterstained in hematoxylin for a minute; with xylene used as permanent mounting medium.

In the context of the present invention, a "control sample" can be any sample that is suitable for a comparison with the sample forming the basis for the method according to the present invention. Control samples can be samples obtained or derived from healthy patients or groups of patients, samples as taken earlier from the same patient or taken from patient or treatment groups.

In the context of the present invention, the term "shortly" shall determine a time period wherein the biological sample is obtained from the subject between directly/immediately after GBM tumor removal until the start of the adjuvant therapy after the GBM tumor removal. Preferred is a shortly as possible after a GBM tumor removal in the subject.

In the context of the present invention, the term "marker" or "biomarker" shall mean a biological molecule, such as a protein or mRNA or detectable fragment thereof, related to the proteins ASAH1, SYNM, GPNBM and MMP-9 as defined herein, that can be detected in a sample in order to determine the expression level of the at least one marker. The markers of the present invention may be in solution or attached to a solid carrier, such as a bead or array. The marker may be suitably labelled, e.g., with an enzymatic, size or weight, antigenic or otherwise detectable label.

"Expression levels" of biomarkers may be numerical values or directions of expression. By "directions" is meant increased or decreased expression, which may be determined as against a control or threshold expression level as explained further herein.

In the context of the present invention, the term "acid ceramidase" shall include both the mammalian, preferably human, protein or a functional fragment thereof, i.e. shall also include stretches and/or regions of the acid ceramidase polypeptide that can be detected. The term also includes the mRNA encoding the protein or a functional fragment thereof. The sequence of human acid ceramidase (ASAH1_HUMAN) can be found in the UniProt database, accession number Q13510. ASAH1 is a lysosomal ceramidase that hydrolyzes sphingolipid ceramides into sphingosine and free fatty acids at acidic pH. Ceramides, sphingosine, and its phosphorylated form sphingosine-1-phosphate are bioactive lipids that mediate cellular signaling pathways regulating several biological processes including cell proliferation, apoptosis and differentiation. It also indirectly regulates tumor necrosis factor/TNF-induced apoptosis.

In the context of the present invention, the term "Synemin" shall include both the mammalian, preferably human, protein or a functional fragment thereof, i.e. shall also include stretches and/or regions of the Synemin that can be detected. The term also includes the mRNA encoding the protein or a functional fragment thereof. The sequence of human Synemin (SYNM_HUMAN) can be found in the UniProt database, accession number 015061. Synemin is a type-VI intermediate filament (IF) which plays an important cytoskeletal role within the muscle cell cytoskeleton. It forms heteromeric IFs with desmin and/or vimentin, and via its interaction with cytoskeletal proteins alpha-dystrobrevin, dystrophin, talin-1, utrophin and vinculin, it is able to link these heteromeric IFs to adherens-type junctions, such as to the costameres, neuromuscular junctions, and myotendinous junctions within striated muscle cells.

In the context of the present invention, the term "Glycoprotein Nmb" shall include both the mammalian, preferably human, protein or a functional fragment thereof, i.e. shall also include stretches and/or regions of the Glycoprotein Nmb that can be detected. The term also includes the mRNA encoding the protein or a functional fragment thereof. The sequence of human Transmembrane glycoprotein NMB (GPNMB _HUMAN) can be found in the UniProt database, accession number Q14956. The protein encoded by this gene is a type I transmembrane glycoprotein which shows homology to the pMEL17 precursor, a melanocyte-specific protein. GPNMB shows expression in the lowly metastatic human melanoma cell lines and xenografts but does not show expression in the highly metastatic cell lines. GPNMB may be involved in growth delay and reduction of metastatic potential. Two transcript variants encoding different isoforms have been found for this gene.

In the context of the present invention, the term "Matrix metalloproteinase-9" shall include both the mammalian, preferably human, protein or a functional fragment thereof, i.e. shall also include stretches and/or regions of the Matrix metalloproteinase-9 polypeptide that can be detected. The term also includes the mRNA encoding the protein or a functional fragment thereof. The sequence of human Matrix metalloproteinase-9 (MMP9_HUMAN) can be found in the UniProt database, accession number P14780. MMPs are a family of zinc-dependent endopeptidases with more than 20 different members. MMP family members can be organized into several groups, such as gelatinases, collagenases, stromelysins, matrilysins and membrane-type MMPs. In particular, MMP-9 plays vital roles in cancer cell invasion and tumor metastasis. focuses on MMP-9, although it is not the only MMP member with vital roles in cancer development. Together with MMP-2, MMP-9 belongs to the gelatinase subgroup of the MMP family.

Preferred in the method for determining the risk for recurrence of glioblastoma multiforme (GBM) in the subject according to the present invention, the subject is selected from the group consisting of a is a cat, dog, mouse, rat, horse, sheep, goat, monkey, or human, preferably a human patient.

Another aspect of the present invention relates to a method for providing a prognosis for the risk for recurrence of glioblastoma multiforme (GBM) in a subject, comprising performing the method according to the present invention, and further comprising the step of providing a prognosis for the subject based on the risk for recurrence as determined, wherein, when compared to a control sample, an increase of *ASAH1* expression level in the biological sample is indicative for a positive prognosis for recurrence of GBM in the subject.

Another aspect of the present invention relates to a method for monitoring the status of glioblastoma multiforme (GBM) in a subject, comprising performing the method according to the present invention, wherein the method is repeated using samples obtained before, during and/or after GBM tumor removal, preferably shortly after GBM tumor removal, more preferably after a first GBM tumor removal, and further comprising the step of monitoring the status of glioblastoma multiforme (GBM) in the subject based on comparing the risk of recurrence as determined.

Another aspect of the invention therefore relates to a method for identifying a subject having glioblastoma multiforme (GBM) in need of adjuvant therapy, comprising performing the method according to the present invention, and further comprising the step of identifying a subject having glioblastoma multiforme (GBM) in need of adjuvant therapy, based on the risk for recurrence as determined.

Preferred in a method according to the present invention, wherein the adjuvant therapy is selected from the group consisting of radiation therapy, chemotherapy, immunotherapy, hormone therapy, and targeted therapy.

Another aspect of the present invention relates to a method for identifying a candidate compound that modulates the time to recurrence of glioblastoma multiforme (GBM) in a subject, comprising contacting a subject having GBM with at least one candidate compound, and determining the expression level of acid ceramidase (*ASAH1*) in a biological sample obtained from the subject, wherein, when compared to a control sample, an increase of expression level of acid ceramidase (*ASAH1*) in the presence of the at least one candidate compound indicates a candidate compound that modulates the time to recurrence, wherein preferably the candidate compound is selected from the group consisting of a chemical molecule, a molecule selected from a library of small organic molecules, a molecule selected from a combinatory library, an anti-cancer chemotherapeutic agent, a cell extract, in particular a plant cell extract, a small molecular drug, a protein, a protein fragment, a molecule selected from a peptide library, and an antibody or fragment thereof.

Yet another aspect of the present invention relates to a method for producing a pharmaceutical composition that is active against a recurrence of glioblastoma multiforme (GBM) in a subject, comprising performing the method according to a method for identifying a candidate compound that modulates the time to recurrence of glioblastoma multiforme (GBM) in a subject and a step of formulating the at least one candidate compound as identified together with a pharmaceutically acceptable carrier, wherein the composition preferably is a tablet, wafer, capsule, granule, powder, sachet, reconstitutable powder, dry powder inhaler and/or chewable.

Such solid formulations may comprise excipients and other ingredients in suitable amounts. Such solid formulations may contain e.g., cellulose, cellulose microcrystalline, polyvidone, in particular FB polyvidone, magnesium stearate and the like. The compound identified as outlined above, which may or may not have gone through additional rounds of modification, is admixed with suitable auxiliary substances and/or additives. Such substances comprise pharmacological acceptable substances, which increase the stability, solubility, biocompatibility, or biological half-life of the interacting compound or comprise substances or materials, which must be included for certain routes of application like, for example, intravenous solution, sprays, liposomes, ointments, skin crème, band-aids or pills.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical compositions of the present invention are employed in co-therapy approaches, i.e. in co-administration with another, or other medicaments or drugs and/or any other therapeutic agent which might be beneficial in the context of the methods of the present invention. Nevertheless, the other pharmaceutical composition of the present invention, medicaments or drugs and/or any other therapeutic agent can be administered separately from the compound as selected or screened and/or compound for use, if required, as long as they act in combination (i.e., directly and/or indirectly, preferably synergistically) with the present compound as selected or screened and/or for use.

Thus, the compounds as selected or screened and/or for use of the invention can be used alone or in combination with other active compounds - for example with medicaments already known for the treatment of bile duct cancer, whereby in the latter case a favorable additive, amplifying or preferably synergistically effect is noticed. Suitable amounts to be administered to humans range from 5 to 500 mg, in particular 10 mg to 100 mg. Of course, any dosage can be readily adjusted by the attending physician, if needed, based on, for example, other medical parameters of the patient to be treated.

Pharmaceutical compositions as used may optionally comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO2), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for respective compounds, for example topical, and will readily be able to choose suitable pharmaceutically acceptable carriers or excipients, depending, e.g., on the formulation and administration route of the pharmaceutical composition.

The therapeutics can be administered orally, e.g., in the form of pills, tablets, coated tablets, sugar coated tablets, hard and soft gelatin capsules, solutions, syrups, emulsions or suspensions or as aerosol mixtures. Administration, however, can also be carried out rectally, e.g., in the form of suppositories, or parenterally, e.g., in the form of injections or infusions, or percutaneously, e.g., in the form of ointments, creams or tinctures.

In addition to the aforementioned compounds as selected or screened and/or for use of the invention, the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more compounds for use of the invention and also other therapeutically active substances as described above.

Thus, yet another aspect of the present invention relates to method for treating or preventing of glioblastoma multiforme (GBM) in a subject, comprising performing the method according to the present invention, and providing a suitable therapy to the subject that is determined as having an increased risk for a relapse of GBM, preferably providing a therapy selected from the group consisting of radiation therapy, chemotherapy, immunotherapy, hormone therapy, and targeted therapy and/or administering an effective amount of the candidate compound as identified according to a method for identifying a candidate compound that modulates the time to recurrence of glioblastoma multiforme (GBM) in a subject or of the pharmaceutical composition as produced.

Another aspect of the invention relates to a candidate compound that is active against a recurrence of glioblastoma multiforme (GBM) in a subject as identified according to the present invention, or the pharmaceutical composition as produced according to the invention for use in the prevention or treatment of recurrence of glioblastoma multiforme (GBM) in a subject.

Yet, another aspect of the invention relates to an array of biomarkers for determining the risk for a recurrence of glioblastoma multiforme (GBM) in a subject according to any one of claims 1 to 5, comprising the biomarker ASAH1 and at least one additional biomarker selected from the group consisting of Synemin (SYNM), Glycoprotein (NMB) (GPNMB), and Matrix Metallopeptidase 9 (MMP-9).

Another aspect of the invention relates to a kit for determining the risk for recurrence of glioblastoma multiforme (GBM) in a subject, comprising materials for performing the method according to the present invention, such as, for example, materials for label-free quantitation (LFQ), buffers, antibodies specifically binding to the biomarker ASAH1, and/or the biomarkers of the above-mentioned array. Optionally, the kit contains additional materials, like a manual or the like. Preferred is the kit according to the present invention, which is suitable for high-throughput screening (HTS).

An additional aspect of the invention relates to the use of the array of biomarkers for determining, providing a prognosis for the risk of recurrence of glioblastoma multiforme (GBM), for monitoring the status of GBM, identifying a subject having GBM in need of adjuvant therapy and/or identifying a candidate compound against the recurrence of GBM.

Yet, another aspect of the invention relates to the use of the kit for determining, providing a prognosis for the risk of recurrence of glioblastoma multiforme (GBM), for monitoring the status of GBM, identifying a subject having GBM in need of adjuvant therapy and/or identifying a candidate compound against the recurrence of GBM.

The present invention relates to the following items:
Item 1: A method for determining the risk for recurrence of glioblastoma multiforme (GBM) in a subject, comprising determining the expression level of acid ceramidase (*ASAH1*) in a biological sample obtained from the subject, wherein an increase of *ASAH1* expression level in the biological sample as determined when compared to a control sample indicates a lower risk for recurrence of GBM in the subject.
Item 2: The method for determining the risk for recurrence of glioblastoma multiforme (GBM) in the subject according to item 1, wherein the subject is a cat, dog, mouse, rat, horse, sheep, goat, monkey, or human, preferably a human patient.
Item 3: The method for determining the risk for recurrence of glioblastoma multiforme (GBM) in the subject according to item 1 or 2 wherein the biological sample is selected from the group consisting of blood, plasma, serum, urine, cerebral spinal fluid, synovial fluid, tumor tissue, and dissociated tumor cells.
Item 4: The method for determining the risk for recurrence of glioblastoma multiforme (GBM) in the subject according to any one of items 1 to 3, wherein the biological sample was obtained from the subject after GBM tumor removal, preferably shortly after GBM tumor removal, more preferably after a first GBM tumor removal.
Item 5: The method for determining the risk for recurrence of glioblastoma multiforme (GBM) in the subject according to any one of items 1 to 4, wherein the determining of the expression level of acid ceramidase (*ASAH1*) in the biological sample comprises a method of determining the amount of *ASAH1* mRNA or ASAH1 protein selected from at least one of ELISA, immunohistochemical (IHC) staining, proteolysis, bicinchoninic acid assay, qPCR and mass spectrometry, preferably further comprising a label-free quantitation (LFQ).
Item 6: A method for providing a prognosis for the risk for recurrence of glioblastoma multiforme (GBM) in a subject, comprising performing the method according to any one of items 1 to 5, and further comprising the step of providing a prognosis for the subject based on the risk for recurrence as determined, wherein, when compared to a control sample, an increase of *ASAH1* expression level in the biological sample is indicative for a positive prognosis for recurrence of GBM in the subject.
Item 7: A method for monitoring the status of glioblastoma multiforme (GBM) in a subject, comprising performing the method according to any one of items 1 to 5, wherein the method is repeated using samples obtained before, during and/or after GBM tumor removal, preferably shortly after GBM tumor removal, more preferably after a first GBM tumor removal, and further comprising the step of monitoring the status of glioblastoma multiforme (GBM) in the subject based on comparing the risk of recurrence as determined.
Item 8: A method for identifying a subject having glioblastoma multiforme (GBM) in need of adjuvant therapy, comprising performing the method according to any one of items 1 to 5, and further comprising the step of identifying a subject having glioblastoma multiforme (GBM) in need of adjuvant therapy, based on the risk for recurrence as determined.
Item 9: The method according to item 8, wherein the adjuvant therapy is selected from the group consisting of radiation therapy, chemotherapy, immunotherapy, hormone therapy, and targeted therapy.
Item 10: The method according to any one of items 6 to 9, wherein the subject is undergoing an anti-cancer therapy, in particular a chemotherapy, surgery, radiation, or androgen ablation.
Item 11 A method for identifying a candidate compound that modulates the time to recurrence of glioblastoma multiforme (GBM) in a subject, comprising contacting a subject having GBM with at least one candidate compound, and determining the expression level of acid ceramidase (*ASAH1*) in a biological sample obtained from the subject, wherein, when compared to a control sample, an increase of expression level of acid ceramidase (*ASAH1*) in the presence of the at least one candidate compound indicates a candidate compound that modulates the time to recurrence, wherein preferably the candidate compound is selected from the group consisting of a chemical molecule, a molecule selected from a library of small organic molecules, a molecule selected from a combinatory library, an anti-cancer chemotherapeutic agent, a cell extract, in particular a plant cell extract, a small molecular drug, a protein, a protein fragment, a molecule selected from a peptide library, and an antibody or fragment thereof.
Item 12: A method for producing a pharmaceutical composition that is active against a recurrence of glioblastoma multiforme (GBM) in a subject, comprising performing the method according to item 11 and a step of formulating the at least one candidate compound as identified together with a pharmaceutically acceptable carrier, wherein the composition preferably is a tablet, wafer, capsule, granule, powder, sachet, reconstitutable powder, dry powder inhaler and/or chewable.
Item 13: A compound as identified according to item 11, or the pharmaceutical composition as produced according to item 12 for use in the prevention or treatment of recurrence of glioblastoma multiforme (GBM) in a subject.
Item 14: A method for treating or preventing of glioblastoma multiforme (GBM) in a subject, comprising performing the method according to any one of items 1 to 5, and providing a suitable therapy to the subject that is determined as having an increased risk for a relapse of GBM, preferably providing a therapy selected from the group consisting of radiation therapy, chemotherapy, immunotherapy, hormone therapy, and targeted therapy and/or administering an effective amount of the candidate compound as identified according to item 11 or of the pharmaceutical composition as produced according to item 12 to the subject.
Item 15: An array of biomarkers for determining the risk for a recurrence of glioblastoma multiforme (GBM) in a subject according to any one of items 1 to 5, comprising the biomarker ASAH1 and at least one additional biomarker selected from the group consisting of Synemin (SYNM), Glycoprotein (NMB) (GPNMB), and Matrix Metallopeptidase 9 (MMP-9).
Item 16: A kit for determining the risk for recurrence of glioblastoma multiforme (GBM) in a subject, comprising materials for performing the method according to any one of items 1 to 11, such as, for example, materials for label-free quantitation (LFQ), buffers, antibodies specifically binding to the biomarker ASAH1, and/or the biomarkers of the array according to item 15.
Item 17: Use of the array of biomarkers according to item 15 for determining, providing a prognosis for the risk of recurrence of glioblastoma multiforme (GBM), for monitoring the status of GBM, identifying a subject having GBM in need of adjuvant therapy and/or identifying a candidate compound against the recurrence of GBM.
Item 18: Use of the kit according to item 16 for determining, providing a prognosis for the risk of recurrence of glioblastoma multiforme (GBM), for monitoring the status of GBM, identifying a subject having GBM in need of adjuvant therapy and/or identifying a candidate compound against the recurrence of GBM.

The invention will now be described further in the following examples with reference to the accompanying figures, nevertheless, without being limited thereto. For the purposes of the present invention, all references as cited are incorporated by reference in their entireties.

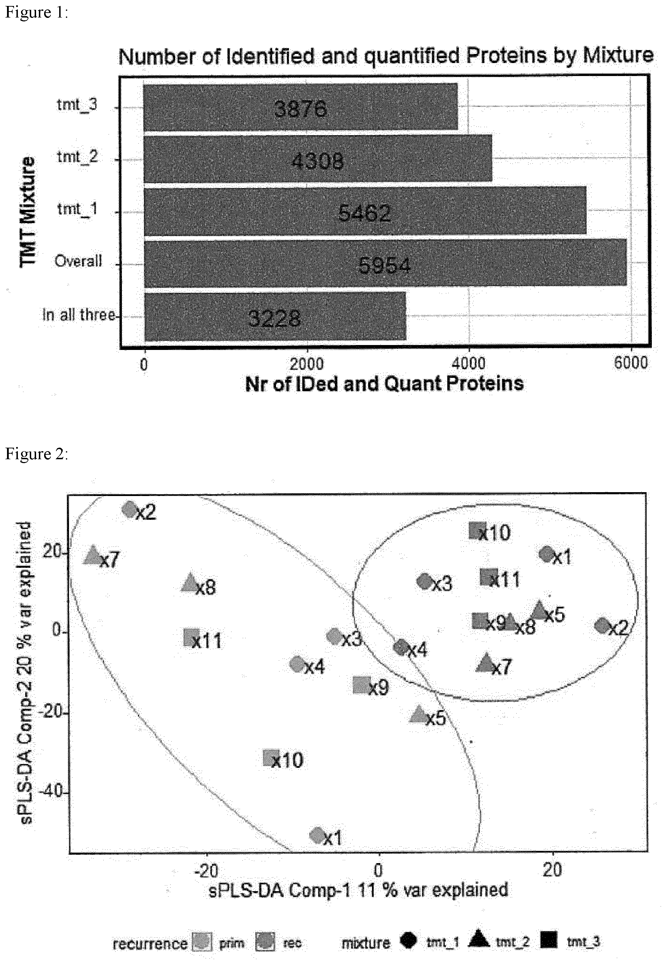

Figure 1 shows a bar graph of the number of identified and quantified proteins by mixture. A total of 3228 overlapping proteins was quantified in all three TMT-11-plex mixture samples. For the tmt-samples, see Figure 2.

Figure 2 shows a sparse least square discriminant analysis (sPLS-DA). The sPLS-DA analysis results in quantitative differences in the proteome between the tumor stages of initial/primary GBM (prim, light gray) and recurrent GBM (rec, dark gray). The tmt-samples were combined as follows: tmt_1: x1, x2, x3, x4; tmt_2: x%, x7, x8; tmt_3: x9, x10, x11.

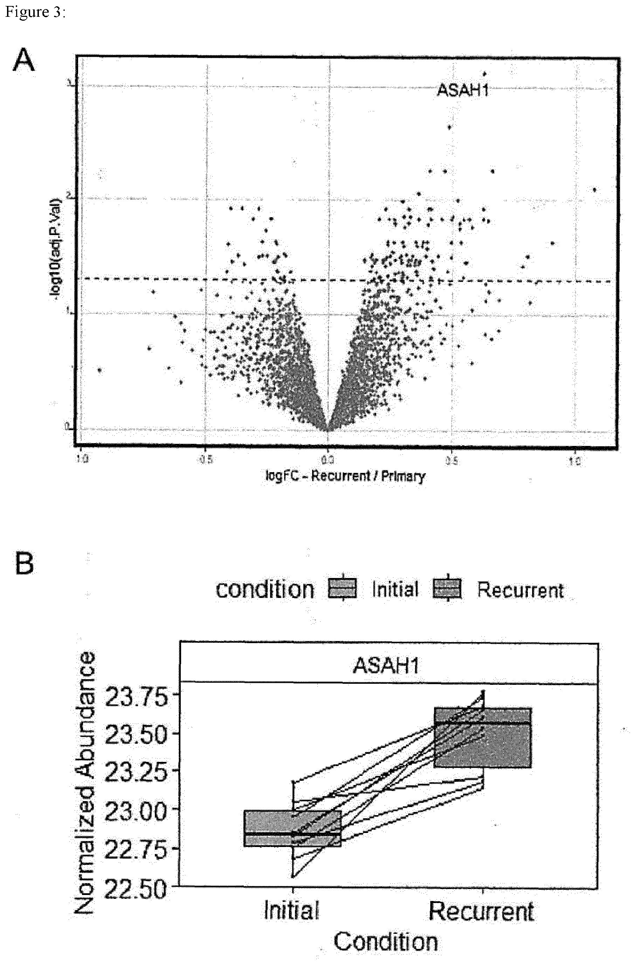

Figure 3 shows the identification of 146 differentially abundant proteins. (A) Volcano plot of differently abundant proteins. (B) Box plots of the normalized differential abundance analysis of ASAH1 in initial GBM (iGBM, light gray) versus recurrent GBM (rGBM, dark gray). The middle line in the box represents the median and the upper and lower ends of the box represent the 75th and 25th percentile, respectively. The minimum and maximum values are also shown.

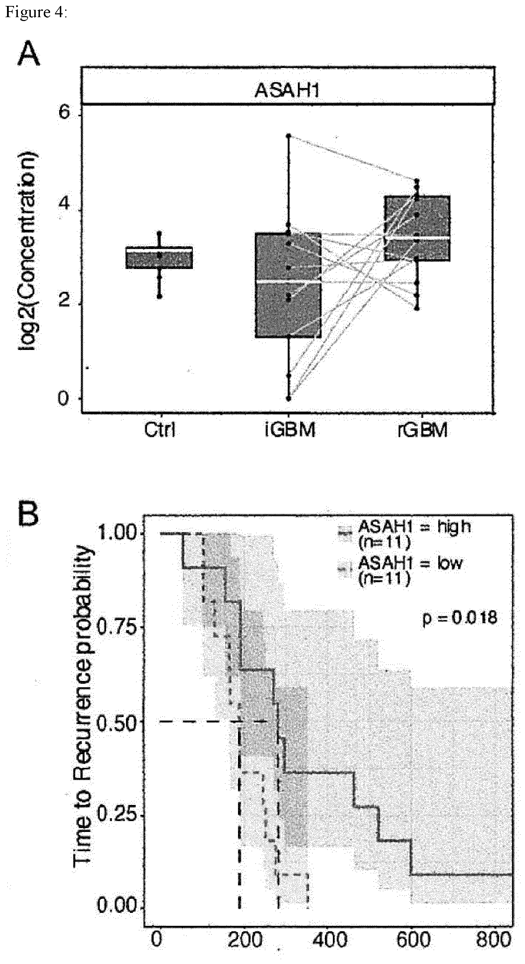

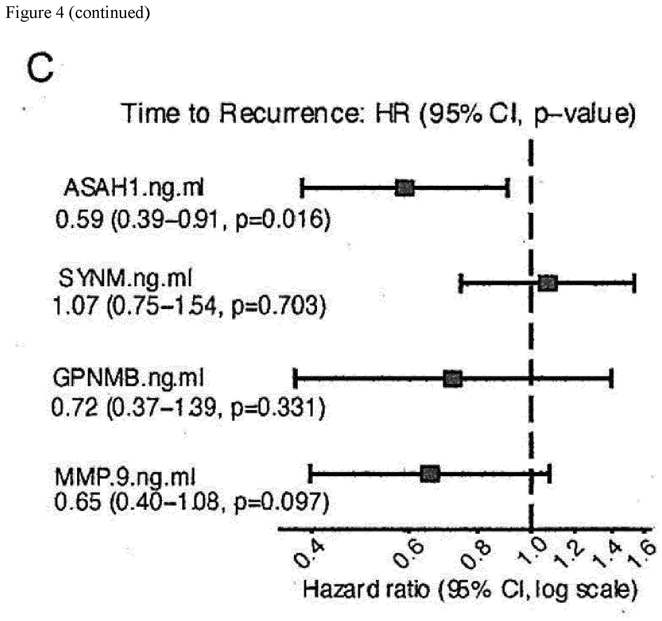

Figure 4 shows the ASAH1 abundances from patient-matched plasma samples taken at the time of initial (iGBM, light gray) and recurrent (rGBM, dark gray) surgery. (A) Comparison of the abundance of ASAH1 in rGBM and iGBM in plasma with a control sample from non-GBM patients as a baseline and mean log 2 concentration (horizontal line). (B) Kaplan-Meier curve of time to recurrence probability for patients with high abundance of ASAH1 and low abundance of ASAH1. n = group of 11 patient-matches samples (C) Forest plot of multi-variate Cox-proportional hazards model of time to recurrence. HR, hazard ratio; 95% CI, confidence interval, log scale.

### Examples

In the context of the present invention, the inventors have investigated a group of 11 patient-matched samples from initial GBM (iGBM) and recurrent (rGBM) glioblastoma tumor specimens, which were surgically removed and stored fresh frozen. Additionally, a total of 30 patient-matched blood plasma samples were collected at iGBM and rGBM time points of resection. The tumor tissue samples were analyzed using liquid chromatography mass spectrometry (LC-MS/MS)-based techniques for a large-scale differential abundance analysis of proteins during iGBM and rGBM. The mass-spectrometry analysis allows the identification of markers associated with the onset of recurrence of GBM, enabling an early prediction about the patient's risk for recurrence and a subsequent adequate treatment. The plasma samples were analyzed via enzyme-linked-immunosorbent-assay (ELISA) for quantification of ASAH1 levels in iGBM, rGBM, and control samples.

The LC-MS/MS analysis was based on the extraction of proteins from tumor tissue after cell lysis performed via ultrasonication and centrifugation. The extracted proteins were reduced with dithiothreitol (DDT), alkylated with iodoacetamide, and digested with Lysyl Endopeptidase (LysC) for two hours and with trypsin overnight. After the digestion, peptides were desalted using PreOmics solid phase columns and vacuum dried. Furthermore, the peptides from each of the samples were then labelled with TMT-11-plex tags and fractionated in an Agilent 1100 HPLC system for increased proteome coverage, using 8 concatenated fractions. The final fractions were measured via an EASY-nLC^{™} 1000 UHPLC system coupled with a Q-Exactive Plus mass spectrometer in DDA mode.

The proteomics analysis yielded in the identification of a total of 5,954 proteins. Based on a multiplexed approach, the inventors identified and consistently quantified 3,228 proteins in every sample from the three TMT-11-plex mixtures (Figure 1).

For all experiments, the inventors performed a statistical analysis for at least three independent experiments employing the unpaired Student's t test using GraphPad Prism 6.0 software (GraphPad Software) with *P* < 0.05 considered significant.

The inventors fit a supervised model based on a sparse least square discriminant analysis (sPLS-DA) to evaluate the quantitative proteome profile for differentiation between the iGBM and the rGBM tumor samples.

sPLS-DA as a sparse version of PLS-DA is a supervised classification context performed well for discriminating and selecting informative variable in one step procedure, which aims to classify a small subset of variables that best recognize the classes (Lê-Cao, Boitard, & Besse, 2011; Rohart, Gautier, Singh, & Lê Cao, 2017). The sPLS-DA segregated the groups indicating the quantitative differences in the proteome between the two tumor stages, iGBM and rGBM (Figure 2).

For the evaluation of quantitative differences in the specific proteins, the inventors used a linear model to explore the association between protein abundance with either iGBM or rGBM, including the patient random effect as a covariate. The inventors accounted for the patient-matched nature of this experimental setup by allowing to explore differences between iGBM and rGBM while accounting for the high inter-patient heterogeneity. The differentially expressed proteins between iGBM and rGBM were selected, according to the criteria of log2 Ifold change| ≥ 1.2 and p < 0.05. This analysis resulted in the identification of 146 differentially abundant proteins, among which 116 were upregulated in rGBM while 30 were found downregulated (Figure 3A).

Among the upregulated proteins in rGBM, ASAH1 was the one showing the smallest adjusted p-value after the statistical testing for differential abundance, indicating a high statistical significance and leading to further statistical analysis. The expression profile of ASAH1 proteomic expression in the tissue samples showed a consistent upregulation in recurrent GBM in every patient. This low variability in the quantitative behavior of ASAH1 explains the smaller adjusted p-value observed via differential abundance analysis (Figure 3B).

Since ASAH1 showed a consistent upregulation in rGBM, the inventors measured the ASAH1 abundances from patient-matched plasma samples taken at the time of initial GBM and recurrent GBM surgery. The measurements were performed via ELISA using plasma samples from non-GBM control patients as a baseline for comparison the general abundance of ASAH1 in plasma. The data showed an increased abundance of ASAH1 in rGBM compared to iGBM (Figure 4A).

For the evaluation whether ASAH1 can be used as a marker for the prediction of a patient's risk for recurrence of GBM at an early stage, a Kaplan-Meier univariate analysis was performed. The analysis showed that higher levels of ASAH1 in plasma at the time of initial surgery are related to a longer time to recurrence probability of the GBM (Figure 4B).

Additionally, a multi-variate Cox-proportional hazards model was applied in which protein abundances were used as continuous variables and a change in hazard ratios (HRs) was assessed, testing their associations with the time of the recurrence of the GBM after the initial surgery. The inventors used plasma levels of SYNM, GPNBM and MMP-9 as variables in the multi-variate model, because these proteins were also observed as differentially abundant in the proteomics analysis. The inventors wanted to evaluate if the association of ASAH1 levels in plasma with longer time to recurrence of the GBM can be caused by a cofounding effect with the abundances of other proteins. Proteins with HR < 1 have more time to recurrence of GBM, while proteins with HR > 1 have less time to recurrence of GBM. The multi-variate model showed consistent results for ASAH1, indicating a significant effect of its plasma levels with longer times to recurrence (Figure 4C).

The above analysis data shows ASAH1 as the most regulated protein between the recurrent and initial tumors. There was a significant increase in abundance in recurrent tumors as well as an increase in plasma levels at the time of recurrent surgery. Based on the data analysis, the inventors concluded that the plasma levels of ASAH1 at the time of the initial surgery are significantly associated with longer times to recurrence. Therefore, ASAH1 plasma-levels can be used as a molecular marker for the time to recurrence of GBM. The blood levels of ASAH1 at the time of the initial surgery can both be used as a prognostic feature and can be used as a marker in GBM diagnostic and treatment schemes.

### References as cited

Doan, N. B. et al., Acid ceramidase and its inhibitors: a de novo drug target and a new class of drugs for killing glioblastoma cancer stem cells with high efficiency. Oncotarget. 8(68), 112662-112674 (2017, Nov).
Doan, N. B. et al., Acid ceramidase confers radioresistance to glioblastoma cells. Oncol Rep. 38(4), 1932-1940 (2017, Oct).
Nguyen, H. S. et al., Molecular Targeting of Acid Ceramidase in Glioblastoma: A Review of Its Role, Potential Treatment, and Challenges. Pharmaceutics. 10(2), 45 (2018).
Hawkins C. C. et al., Targeting Acid Ceramidase Inhibits Glioblastoma Cell Migration through Decreased AKT Signaling. Cells. 11(12), 1873 (2022).
Lê Cao, K.-A., Boitard, K., & Besse, S. (2011). Sparse PLS discriminant analysis: Biologically relevant feature selection and graphical displays for multiclass problems. BMC Bioinformatics, 12(1), 1-16.
Rohart, F., Gautier, B., Singh, A., & Lê Cao, K. (2017). MixOmics: An R package for 'omics feature selection and multiple data integration. PloS Computational Biology, 13(11), e1005752

## Claims

1. A method for determining the risk for recurrence of glioblastoma multiforme (GBM) in a subject, comprising:
determining the expression level of acid ceramidase (*ASAH1*) in a biological sample obtained from the subject,
wherein an increase of *ASAH1* expression level in the biological sample as determined when compared to a control sample indicates a lower risk for recurrence of GBM in the subject, wherein preferably the subject is a cat, dog, mouse, rat, horse, sheep, goat, monkey, or human, preferably a human patient suffering from GBM.

2. The method according to claim 1, wherein the biological sample is selected from the group consisting of blood, plasma, serum, urine, cerebral spinal fluid, synovial fluid, tumor tissue, and dissociated tumor cells.

3. The method according to claim 1 or 2, wherein the biological sample was obtained from the subject after GBM tumor removal, preferably shortly after GBM tumor removal, more preferably after a first GBM tumor removal.

4. The method according to any one of claims 1 to 3, wherein the determining of the expression level of acid ceramidase (*ASAH1*) in the biological sample comprises a method of determining the amount of *ASAH1* mRNA or ASAH1 protein selected from at least one of ELISA, immunohistochemical (IHC) staining, proteolysis, bicinchoninic acid assay, qPCR and mass spectrometry, preferably further comprising a label-free quantitation (LFQ).

5. A method for providing a prognosis for the risk for recurrence of glioblastoma multiforme (GBM) in a subject, comprising:
performing the method according to any one of claims 1 to 4, and
further comprising the step of providing a prognosis for the subject based on the risk for recurrence as determined, wherein, when compared to a control sample, an increase of *ASAH1* expression level in the biological sample is indicative for a positive prognosis for recurrence of GBM in the subject.

6. A method for monitoring the status of glioblastoma multiforme (GBM) in a subject, comprising:
performing the method according to any one of claims 1 to 4, wherein the method is repeated using samples obtained before, during and/or after GBM tumor removal, preferably shortly after GBM tumor removal, more preferably after a first GBM tumor removal,
and
further comprising the step of monitoring the status of glioblastoma multiforme (GBM) in the subject based on comparing the risk of recurrence as determined.

7. A method for identifying a subject having glioblastoma multiforme (GBM) in need of adjuvant therapy, comprising:
performing the method according to any one of claims 1 to 4, and
further comprising the step of identifying a subject having glioblastoma multiforme (GBM) in need of adjuvant therapy, based on the risk for recurrence as determined, wherein preferably the adjuvant therapy is selected from the group consisting of radiation therapy, chemotherapy, immunotherapy, hormone therapy, and targeted therapy.

8. The method according to any one of claims 5 to 7, wherein the subject is undergoing an anti-cancer therapy, in particular a chemotherapy, surgery, radiation, or androgen ablation.

9. A method for identifying a candidate compound that modulates the time to recurrence of glioblastoma multiforme (GBM) in a subject, comprising:
contacting a subject having GBM with at least one candidate compound, and
determining the expression level of acid ceramidase (*ASAH1*) in a biological sample obtained from the subject,
wherein, when compared to a control sample, an increase of expression level of acid ceramidase (*ASAH1*) in the presence of the at least one candidate compound indicates a candidate compound that modulates the time to recurrence, wherein preferably the candidate compound is selected from the group consisting of a chemical molecule, a molecule selected from a library of small organic molecules, a molecule selected from a combinatory library, an anti-cancer chemotherapeutic agent, a cell extract, in particular a plant cell extract, a small molecular drug, a protein, a protein fragment, a molecule selected from a peptide library, and an antibody or fragment thereof.

10. A method for producing a pharmaceutical composition that is active against a recurrence of glioblastoma multiforme (GBM) in a subject, comprising:
performing the method according to claim 9 and furthermore the step of formulating the at least one candidate compound as identified together with a pharmaceutically acceptable carrier, wherein the composition preferably is a tablet, wafer, capsule, granule, powder, sachet, reconstitutable powder, dry powder inhaler and/or chewable.

11. A compound as identified according to claim 9, or the pharmaceutical composition as produced according to claim 10 for use in the prevention or treatment of recurrence of glioblastoma multiforme (GBM) in a subject.

12. An array of biomarkers for determining the risk for a recurrence of glioblastoma multiforme (GBM) in a subject according to any one of claims 1 to 4, comprising the biomarker ASAH1 and at least one additional biomarker selected from the group consisting of Synemin (SYNM), Glycoprotein (NMB) (GPNBM), and Matrix Metallopeptidase 9 (MMP-9).

13. A kit for determining the risk for recurrence of glioblastoma multiforme (GBM) in a subject, comprising materials for performing the method according to any one of claims 1 to 9, such as, for example, materials for label-free quantitation (LFQ), buffers, antibodies specifically binding to the biomarker ASAH1, and/or the biomarkers of the array according to claim 12.

14. Use of the array of biomarkers according to claim 12 for determining, providing a prognosis for the risk of recurrence of glioblastoma multiforme (GBM), for monitoring the status of GBM, identifying a subject having GBM in need of adjuvant therapy and/or identifying a candidate compound against the recurrence of GBM.

15. Use of the kit according to claim 13 for determining, providing a prognosis for the risk of recurrence of glioblastoma multiforme (GBM), for monitoring the status of GBM, identifying a subject having GBM in need of adjuvant therapy and/or identifying a candidate compound against the recurrence of GBM.
